(19) Europäisches Patentamt
European Patent Office
Office européen des brevets

(11) **EP 3 943 894 A1**

(12) **EUROPÄISCHE PATENTANMELDUNG**

(43) Veröffentlichungstag:
26.01.2022  Patentblatt 2022/04

(21) Anmeldenummer: 21186368.3

(22) Anmeldetag: **19.07.2021**

(51) Internationale Patentklassifikation (IPC):
**G01F 7/00** (2006.01)       **A61B 1/12** (2006.01)
**A61B 90/70** (2016.01)      G01F 1/06 (2006.01)
G01F 1/10 (2006.01)          G01F 1/34 (2006.01)
G01F 1/68 (2006.01)          G01F 3/10 (2006.01)

(52) Gemeinsame Patentklassifikation (CPC):
**G01F 7/00; A61B 1/123; A61B 1/125; A61B 90/70;**
G01F 1/06; G01F 1/10; G01F 1/34; G01F 1/68;
G01F 3/10

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **23.07.2020  DE 102020119416**

(71) Anmelder: **Miele & Cie. KG**
**33332 Gütersloh (DE)**

(72) Erfinder: **Werner, Alexander**
**33619 Bielefeld (DE)**

(54) **SENSOREINHEIT, REINIGUNGSGERÄT FÜR MEDIZINISCHES REINIGUNGSGUT UND VERFAHREN ZUM STEUERN EINER SENSOREINHEIT**

(57)     Die Erfindung betrifft eine Sensoreinheit (1) zum Bestimmen eines Volumenstroms eines Fluides für einen Reinigungsvorgang von medizinischem Reinigungsgut. Die Sensoreinheit (1) umfasst einen Eingangsanschluss (E) zum Einleiten des Fluides in die Sensoreinheit (1), einen Ausgangsanschluss (A) zum Ausleiten des Fluides aus der Sensoreinheit (1) und einen Fluidkanal mit einem ersten Fluidflusspfad (F0) und mit einem zu dem ersten Fluidflusspfad (F0) strömungsmechanisch parallelen, zweiten Fluidflusspfad (F1). Ein erster Durchflusssensor (S0) ist ausgebildet, um im Rahmen eines ersten Messbereichs den Volumenstrom in dem ersten Fluidflusspfad (F0) zu erfassen und ein erstes Sensorsignal (y0) bereitzustellen. Ein zweiter Durchflusssensor (S1) ist ausgebildet, um im Rahmen eines zweiten Messbereichs den Volumenstrom in dem zweiten Fluidflusspfad (F1) zu erfassen und ein zweites Sensorsignal (y1) bereitzustellen. Eine Steuereinheit (CPU) ist ausgebildet, um abhängig von zumindest einem der Sensorsignale (y0, y1) ein Ventil (V1) zum Freigeben und Sperren zumindest eines der Fluidflusspfade (F0, F1) anzusteuern, sowie abhängig von zumindest einem der Sensorsignale (y0, y1) den Volumenstrom des Fluids zu bestimmen.

FIG 1

**Beschreibung**

Die Erfindung betrifft eine Sensoreinheit, ein Reinigungsgerät für medizinisches Reinigungsgut und durch ein Verfahren zum Steuern einer Sensoreinheit.

Zur Durchflussmessung bzw. Volumenstrommessung von Gasen und/oder anderen Fluiden können z. B. im Messzweig verbaute Flügelradzähler, Ovalradzähler oder kalorimetrisch wirkende Durchflussmesser eingesetzt werden. Als weitere Möglichkeit einer Durchflussmessung kann auch das sogenannte Differenzdruckverfahren angewendet werden, das den Durchfluss indirekt über die Druckdifferenz einer im Strang verbauten Blende erkennen kann. Insbesondere können bei der Durchflussmessung von strömenden Gasen Durchflussmesser bzw. Volumenstrommesser oder Massenflussmesser eingesetzt werden. Je nach Anwendungsfall sollen mit einer Messvorrichtung beispielsweise verschiedene Volumenströme hinreichend genau erkannt werden. Sind bei einer Anwendung Volumenströme in einem breiten Lösungsraum zu erwarten, wobei eine Messvorrichtung sowohl langsam strömende als auch schnell strömende Volumenströme gleichermaßen gut erkennen soll, so sollte ein in der Messvorrichtung verwendeter Sensor über den gesamten Messbereich exakte und hochauflösende Werte bereitstellen. Solche Systeme können in vielen Anwendungen insbesondere aus Kostengründen nicht angewendet werden, weshalb häufig Kompromisslösungen genutzt werden, wobei zum Beispiel ein Sensor im mittleren Messbereich vergleichsweise gut funktioniert, aber beim Nulldurchgang und in Richtung des Maximums des Messbereichs ungenauere Werte liefern kann.

Der Erfindung stellt sich die Aufgabe, eine verbesserte Sensoreinheit, ein verbessertes Reinigungsgerät für medizinisches Reinigungsgut und ein verbessertes Verfahren zum Steuern einer Sensoreinheit bereitzustellen.

Erfindungsgemäß wird diese Aufgabe durch eine Sensoreinheit, durch ein Reinigungsgerät und durch ein Verfahren mit den Merkmalen der Hauptansprüche gelöst. Vorteilhafte Ausgestaltungen und Weiterbildungen der Erfindung ergeben sich aus den nachfolgenden Unteransprüchen.

Die mit der Erfindung erreichbaren Vorteile bestehen insbesondere darin, dass eine kaskadierte und/oder zwei- oder mehrstufige Durchflussmessung von strömenden Fluiden, beispielsweise von strömenden Gasen, bereitgestellt werden kann, wobei eine Inline-Volumenstrommessung von Gasen in einem unbegrenzt weit gefassten Messbereich mit hohen Empfindlichkeiten für verschiedene Intervalle des gesamten Messbereichs und/oder eine mehrstufige Durchflussmessung mit interner automatischer Wahl des am besten geeigneten Messintervalls realisiert werden kann. So kann beispielsweise eine sehr präzise Messung von langsam strömenden Fluiden und hinreichend präzise Messung von schnell strömenden Fluiden in nur einer Messvorrichtung bzw. Sensoreinheit ermöglicht werden. Es kann eine Anwendung für jeden beliebigen Messbereich erzielt werden. Für einen Anwender kann die Sensoreinheit wie ein einzelner Sensor behandelt werden, der inline verbaut werden kann und dem Anwender bzw. einer Recheneinheit stets ein einziges Ausgangssignal liefert, unabhängig von einem derzeit aktiven Intervall des Messbereichs, den die Sensoreinheit abdeckt. Es kann eine Sensoreinheit mit einer kompakten Bauweise für einen breit gefächerten Anwendungsbereich und/oder Messbereich bereitgestellt werden. Anders als insbesondere bei einer Verwendung von einem einzelnen beispielsweise kalorimetrisch wirkenden Durchflusssensor kann vermieden werden, dass eine Entscheidung zwischen einem Sensor mit großem Messbereich und tendenziell geringer Empfindlichkeit und einem Sensor mit kleinerem Messbereich und meist höherer Empfindlichkeit zu treffen ist. So kann auch verhindert werden, dass wie bei dem letztgenannten Sensor durch seinen Aufbau und seine Charakteristik ein maximaler Volumenstrom, der durch den Sensor geleitet werden kann, gedrosselt würde. Es können gemäß Ausführungsformen in zumindest zwei Messzweigen bzw. Messsträngen sowohl kleine Volumenströme sehr genau gemessen werden und zudem auch deutlich höhere Volumenströme zugelassen und ebenfalls gemessen werden.

Es wird eine Sensoreinheit zum Bestimmen eines Volumenstroms eines Fluides für einen Reinigungsvorgang von medizinischem Reinigungsgut vorgestellt, wobei die Sensoreinheit folgende Merkmale aufweist:

einen Eingangsanschluss zum Einleiten des Fluides in die Sensoreinheit, einen Ausgangsanschluss zum Ausleiten des Fluides aus der Sensoreinheit und einen Fluidkanal mit einem ersten Fluidflusspfad und mit einem zu dem ersten Fluidflusspfad strömungsmechanisch parallelen, zweiten Fluidflusspfad, wobei der Eingangsanschluss und der Ausgangsanschluss über den Fluidkanal strömungsmechanisch miteinander verbunden sind;

einen ersten Durchflusssensor mit einem ersten Messbereich und einen zweiten Durchflusssensor mit einem von dem ersten Messbereich unterschiedlichen, zweiten Messbereich, wobei der erste Durchflusssensor ausgebildet ist, um im Rahmen des ersten Messbereichs den Volumenstrom in dem ersten Fluidflusspfad zu erfassen und ein erstes Sensorsignal bereitzustellen, das erste Messwerte des erfassten Volumenstroms in dem ersten Fluidflusspfad repräsentiert, wobei der zweite Durchflusssensor ausgebildet ist, um im Rahmen des zweiten Messbereichs den Volumenstrom in dem zweiten Fluidflusspfad zu erfassen und ein zweites Sensorsignal bereitzustellen, das zweite Messwerte des erfassten Volumenstroms in dem zweiten Fluidflusspfad repräsentiert;

zumindest ein Ventil, das zum Freigeben und Sperren zumindest eines der Fluidflusspfade für einen Durchfluss des Fluides mittels eines Steuersignals ansteuerbar ist; und

eine Steuereinheit, die ausgebildet ist, um abhängig von zumindest einem der Sensorsignale das Steuersignal bereitzustellen, und abhängig von zumindest einem der Sensorsignale den Volumenstrom des Fluids zu bestimmen und ein den Volumenstrom des Fluids repräsentierendes Ausgangssignal bereitzustellen.

Bei dem Fluid kann es sich um ein Gas oder gasförmiges Medium handeln, insbesondere um Druckluft. Der erste Messbereich und der zweite Messbereich können einen gesamten Messbereich der Sensoreinheit ergeben bzw. definieren. Mittels des Steuersignals kann das zumindest eine Ventil aktiviert, deaktiviert oder umgeschaltet werden.

Insbesondere können die Durchflusssensoren Volumenstromsensoren und zusätzlich oder alternativ Massenstromsensoren aufweisen. Zusätzlich oder alternativ kann zumindest einer der Durchflusssensoren in dem ersten Fluidflusspfad oder in dem zweiten Fluidflusspfad angeordnet sein. Zusätzlich oder alternativ kann zumindest einer der Durchflusssensoren einen Flügelradzähler, einen Ovalradzähler, einen Differenzdrucksensor oder einen kalorimetrisch wirkenden Durchflusssensor aufweisen. Somit kann die Sensoreinheit flexibel und genau für jeweils vorliegende Bedürfnisse und gewünschte Messbereiche ausgelegt werden.

Gemäß einer Ausführungsform kann der erste Messbereich kleiner als der zweite Messbereich sein. Zusätzlich oder alternativ können sich hierbei die Messbereiche zumindest teilweise überlappen. Zusätzlich oder alternativ kann die Steuereinheit ausgebildet sein, um das zumindest eine Ventil anzusteuern, um den zweiten Fluidflusspfad zu sperren, wenn die ersten Messwerte unterhalb eines für den ersten Messbereich gültigen, ersten oberen Schwellenwertes liegen oder die zweiten Messwerte unterhalb eines für den zweiten Messbereich gültigen, zweiten unteren Schwellenwertes liegen. Eine solche Ausführungsform bietet den Vorteil, dass insbesondere bei einer hohen Wahrscheinlichkeit für Volumenströme in einem engen Wertebereich eine einfache Anwendung realisiert werden kann.

Gemäß einer anderen Ausführungsform kann der erste Messbereich größer als der zweite Messbereich sein. Zusätzlich oder alternativ können sich hierbei die Messbereiche zumindest teilweise überlappen. Zusätzlich oder alternativ kann die Steuereinheit ausgebildet sein, um das zumindest eine Ventil anzusteuern, um den zweiten Fluidflusspfad zu sperren, wenn der durch das Ausgangssignal repräsentierte Volumenstrom oberhalb eines für den ersten Messbereich gültigen, ersten unteren Schwellenwertes oder oberhalb eines für den zweiten Messbereich gültigen, zweiten oberen Schwellenwertes liegt. Eine solche Ausführungsform bietet den Vorteil, dass auch für stark variierende Volumenströme deren Bestimmung auf einfache Weise erzielt werden kann.

Auch kann das zumindest eine Ventil in dem zweiten Fluidflusspfad angeordnet sein. Zusätzlich oder alternativ kann das zumindest eine Ventil in Fluidflussrichtung stromabwärts des zweiten Durchflusssensors angeordnet sein. Zusätzlich oder alternativ kann das zumindest eine Ventil als ein normalerweise geschlossenes Ventil oder normalerweise geschlossenes 2/2-Wege-Ventil ausgeführt sein. So kann ein besonders einfacher Aufbau insbesondere auch für mehrere Fluidflusspfade erreicht werden.

Alternativ kann das zumindest eine Ventil zwischen dem Eingangsanschluss und einer Verzweigungsstelle des Fluidkanals in den ersten Fluidflusspfad und den zweiten Fluidflusspfad angeordnet sein. Zusätzlich oder alternativ kann das zumindest eine Ventil in Fluidflussrichtung stromaufwärts der Durchflusssensoren angeordnet sein. Zusätzlich oder alternativ kann das zumindest eine Ventil als ein 3/2-Wege-Ventil ausgeführt sein. Auf diese Weise kann zuverlässig zwischen zwei Fluidflusspfaden umgeschaltet werden oder zumindest ein Fluidflusspfad freigegeben werden.

Dabei kann gemäß einer Ausführungsform der erste Durchflusssensor einen Differenzdrucksensor aufweisen und kann der zweite Durchflusssensor einen in dem zweiten Fluidflusspfad angeordneten Volumenstromsensor aufweisen.

Ferner kann die Steuereinheit ausgebildet sein, um den Volumenstrom durch Mittelwertbildung unter Verwendung einer Mehrzahl von Messwerten zu bestimmen. Somit kann ein Hystereseflackern bei einer Berechnungsroutine des Ausgangssignals bzw. Ausgabewertes durch Mittelwertbildung der Sensorsignale erreicht werden.

Zudem kann die Sensoreinheit zumindest einen weiteren Fluidflusspfad, zumindest einen weiteren Durchflusssensor und zumindest ein weiteres Ventil aufweisen. Hierbei kann der zumindest eine weitere Fluidflusspfad strömungsmechanisch parallel zu dem ersten Fluidflusspfad und dem zweiten Fluidflusspfad angeordnet sein. Der zumindest eine weitere Durchflusssensor kann ausgebildet sein, um im Rahmen zumindest eines von dem ersten Messbereich und dem zweiten Messbereich unterschiedlichen, weiteren Messbereichs den Volumenstrom in dem zumindest einen weiteren Fluidflusspfad zu erfassen und zumindest ein weiteres Sensorsignal bereitzustellen, das weitere Messwerte des erfassten Volumenstroms in dem zumindest einen weiteren Fluidflusspfad repräsentiert. Das zumindest eine weitere Ventil kann zum Freigeben und Sperren des zumindest einen weiteren Fluidflusspfads ansteuerbar sein. Eine einzige Sensoreinheit kann somit als Messvorrichtung für eine beliebige Anzahl an zu messenden Kanälen verwendet werden, die insbesondere separat aktiv geschaltet werden können.

Es wird auch ein Reinigungsgerät für medizinisches Reinigungsgut vorgestellt, wobei das Reinigungsgerät folgende Merkmale aufweist:

einen Spülraum zum Aufnehmen des Reinigungsguts, wobei der Spülraum zumindest eine Fluidschnittstelle zur strömungsmechanischen Kopplung mit dem Reinigungsgut aufweist;

eine Bereitstellungseinrichtung zum Bereitstellen von Druckluft, wobei die Bereitstellungseinrichtung strömungsmechanisch mit der zumindest einen Fluidschnittstelle verbunden ist; und

eine Ausführungsform der vorstehend genannten Sensoreinheit, wobei die Sensoreinheit strömungsmechanisch zwischen die Bereitstellungseinrichtung und die zumindest eine Fluidschnittstelle geschaltet ist.

In Verbindung mit dem Reinigungsgerät kann eine Ausführungsform der vorstehend genannten Sensoreinheit vorteilhaft eingesetzt oder verwendet werden, um einen Volumenstrom der Druckluft zu bestimmen.

Das Reinigungsgerät kann verwendet werden, um einen Reinigungs- und Desinfektionsprozess von englumigen, medizinischen Spülgütern durchzuführen. Dazu kann eine Drucklufteinspeisung beim Reinigungsschritt oder Trocknungsschritt erfolgen.

Somit kann die zumindest eine Fluidschnittstelle zum Koppeln des Reinigungsguts in Form von englumigen, medizinischen Spülgütern ausgeformt sein. Beispielsweise können dadurch auch Endoskope gereinigt und desinfiziert werden.

Die Bereitstellungseinrichtung kann zum Bereitstellen der Druckluft zur Drucklufteinspeisung zum Reinigen oder Trocknen des Reinigungsguts ausgebildet sein. Die Drucklufteinspeisung kann dabei vorteilhafterweise unter Verwendung der Sensoreinheit überwacht und dadurch auch gesteuert werden.

Es wird ferner ein Verfahren zum Steuern einer Ausführungsform der vorstehend genannten Sensoreinheit vorgestellt, wobei das Verfahren folgende Schritte aufweist:
Einlesen zumindest eines der Sensorsignale von zumindest einem der Durchflusssensoren;

Verarbeiten des zumindest einen Sensorsignals, um den Volumenstrom zu bestimmen, das Ausgangssignal zu erzeugen und das Steuersignal zu generieren; und

Bereitstellen des Steuersignals zur Ausgabe an das zumindest eine Ventil und des Ausgangssignals.

Das Verfahren zum Steuern ist mittels der Steuereinheit einer Ausführungsform der vorstehend genannten Sensoreinheit vorteilhaft ausführbar, um die Sensoreinheit zu steuern.

Die hier beschriebene Vorrichtung und/oder das hier beschriebene Verfahren kann bzw. können im Zusammenhang mit einem gewerblichen oder professionellen Gerät, beispielsweise einem medizinischen Gerät, wie einem Reinigungs- oder Desinfektionsgerät, einem Kleinsterilisator, einem Großraumdesinfektor oder einer Container-Waschanlage eingesetzt werden.

Der hier vorgestellte Ansatz schafft ferner eine Vorrichtung oder Steuereinheit, die ausgebildet ist, um die Schritte einer Variante eines hier vorgestellten Verfahrens in entsprechenden Einrichtungen durchzuführen, anzusteuern bzw. umzusetzen. Auch durch diese Ausführungsvariante der Erfindung in Form einer Vorrichtung oder Steuereinheit kann die der Erfindung zugrunde liegende Aufgabe schnell und effizient gelöst werden. Bei der Steuereinheit oder Vorrichtung kann es sich um die Steuereinheit der Sensoreinheit handeln.

Die Vorrichtung oder Steuereinheit kann ausgebildet sein, um Eingangssignale einzulesen und unter Verwendung der Eingangssignale Ausgangssignale zu bestimmen und bereitzustellen. Ein Eingangssignal kann beispielsweise ein über eine Eingangsschnittstelle der Vorrichtung oder Steuereinheit einlesbares Sensorsignal darstellen. Ein Ausgangssignal kann ein Steuersignal oder ein Datensignal darstellen, das an einer Ausgangsschnittstelle der Vorrichtung bereitgestellt werden kann. Die Vorrichtung oder Steuereinheit kann ausgebildet sein, um die Ausgangssignale unter Verwendung einer in Hardware oder Software umgesetzten Verarbeitungsvorschrift zu bestimmen. Beispielsweise kann die Vorrichtung oder Steuereinheit dazu eine Logikschaltung, einen integrierten Schaltkreis oder ein Softwaremodul umfassen und beispielsweise als ein diskretes Bauelement realisiert sein oder von einem diskreten Bauelement umfasst sein.

Von Vorteil ist auch ein Computer-Programmprodukt oder Computerprogramm mit Programmcode, der auf einem maschinenlesbaren Träger oder Speichermedium wie einem Halbleiterspeicher, einem Festplattenspeicher oder einem optischen Speicher gespeichert sein kann. Wird das Programmprodukt oder Programm auf einem Computer oder einer Vorrichtung oder Steuereinheit ausgeführt, so kann das Programmprodukt oder Programm zur Durchführung, Umsetzung und/oder Ansteuerung der Schritte des Verfahrens nach einer der vorstehend beschriebenen Ausführungsformen verwendet werden.

Ein Ausführungsbeispiel der Erfindung ist in den Zeichnungen rein schematisch dargestellt und wird nachfolgend näher beschrieben. Es zeigt

| Figur 1 | eine schematische Darstellung einer Sensoreinheit gemäß einem Ausführungsbeispiel; |
| Figur 2 | ein exemplarisches Volumenstrom-Amplitude-Diagramm gemäß einem Ausführungsbeispiel; |
| Figur 3 | eine schematische Darstellung einer Sensoreinheit gemäß einem Ausführungsbeispiel; |
| Figur 4 | eine schematische Darstellung einer Sensoreinheit gemäß einem Ausführungsbeispiel; |
| Figur 5 | eine schematische Darstellung einer Sensoreinheit gemäß einem Ausführungsbeispiel; |

Figur 6    eine schematische Darstellung einer Steuereinheit gemäß einem Ausführungsbeispiel;

Figur 7    ein Ablaufdiagramm eines Messverfahrens gemäß einem Ausführungsbeispiel;

Figur 8    eine schematische Darstellung einer Sensoreinheit gemäß einem Ausführungsbeispiel;

Figur 9    ein Ablaufdiagramm eines Messverfahrens gemäß einem Ausführungsbeispiel;

Figur 10    eine schematische Darstellung eines Reinigungsgeräts gemäß einem Ausführungsbeispiel;

Figur 11    eine schematische Darstellung einer Sensoreinheit gemäß einem Ausführungsbeispiel; und

Figur 12    ein Ablaufdiagramm eines Verfahrens zum Steuern gemäß einem Ausführungsbeispiel.

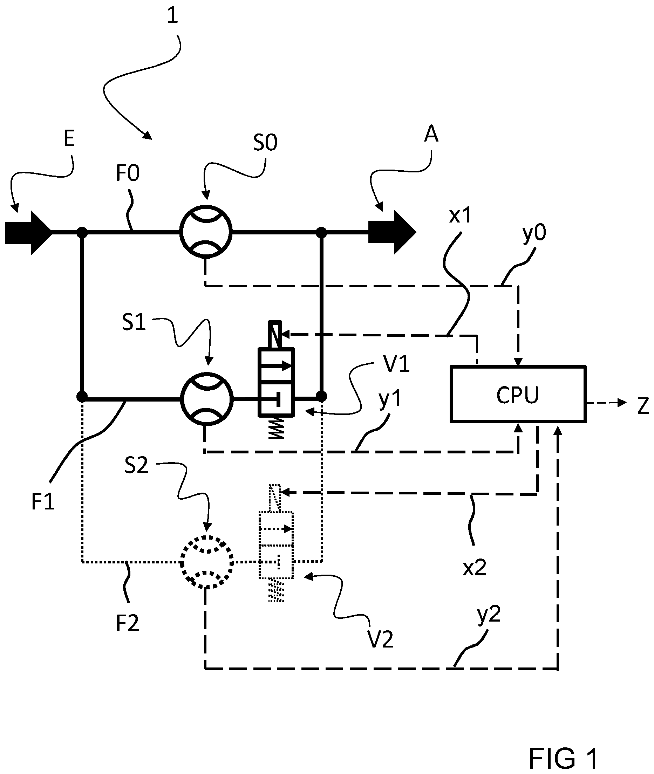

Figur 1 zeigt eine schematische Darstellung einer Sensoreinheit 1 gemäß einem Ausführungsbeispiel. Die Sensoreinheit 1 ist ausgebildet, um einen Volumenstrom eines Fluides für einen Reinigungsvorgang von medizinischem Reinigungsgut, wie zum Beispiel zumindest eines Endoskops, zu bestimmen.

Die Sensoreinheit 1 umfasst ein Eingangsanschluss E zum Einleiten des Fluides in die Sensoreinheit 1, einen Ausgangsanschluss A zum Ausleiten des Fluides aus der Sensoreinheit 1 und einen Fluidkanal mit einem ersten Fluidflusspfad F0 bzw. ersten Strang oder Initialstrang und mit einem zu dem ersten Fluidflusspfad F0 strömungsmechanisch parallelen, zweiten Fluidflusspfad F1. Der Eingangsanschluss E und der Ausgangsanschluss A sind über den Fluidkanal strömungsmechanisch miteinander verbunden.

Die Sensoreinheit 1 umfasst ferner einen ersten Durchflusssensor S0 mit einem ersten Messbereich bzw. Messintervall und einen zweiten Durchflusssensor S1 mit einem von dem ersten Messbereich unterschiedlichen, zweiten Messbereich bzw. Messintervall. Der erste Durchflusssensor S0 ist ausgebildet, um im Rahmen des ersten Messbereichs den Volumenstrom in dem ersten Fluidflusspfad F0 zu erfassen und ein erstes Sensorsignal y0 bereitzustellen, das erste Messwerte des erfassten Volumenstroms in dem ersten Fluidflusspfad F0 repräsentiert. Der zweite Durchflusssensor S1 ist ausgebildet, um im Rahmen des zweiten Messbereichs den Volumenstrom in dem zweiten Fluidflusspfad F1 zu erfassen und ein zweites Sensorsignal y1 bereitzustellen, das zweite Messwerte des erfassten Volumenstroms in dem zweiten Fluidflusspfad F1 repräsentiert.

Zudem umfasst die Sensoreinheit 1 auch zumindest ein Ventil V1, das zum Freigeben und Sperren zumindest eines der Fluidflusspfade F0, F1 für einen Durchfluss des Fluides ansteuerbar ist. Die Sensoreinheit 1 umfasst auch eine Steuereinheit CPU, die ausgebildet ist, um abhängig von zumindest einem der Sensorsignale y0, y1 mittels eines Steuersignals x1 das zumindest eine Ventil V1 anzusteuern. Die Steuereinheit CPU ist ferner ausgebildet, um abhängig von zumindest einem der Sensorsignale y0, y1 den Volumenstrom zu bestimmen. Auch ist die Steuereinheit CPU ausgebildet, um ein den bestimmten Volumenstrom repräsentierendes Ausgangssignal Z bereitzustellen.

Gemäß dem hier dargestellten Ausführungsbeispiel ist das zumindest eine Ventil V1 in dem zweiten Fluidflusspfad F1 angeordnet. Dabei ist das zumindest eine Ventil V1 in Fluidflussrichtung bzw. Flussrichtung des Fluides stromabwärts des zweiten Durchflusssensors S1 angeordnet. Insbesondere ist hierbei das zumindest eine Ventil V1 als ein normalerweise geschlossenes Ventil, genauer gesagt als ein normalerweise geschlossenes 2/2-Wege-Ventil ausgeführt.

Ferner umfasst die Sensoreinheit 1 gemäß einem Ausführungsbeispiel zumindest einen weiteren Fluidflusspfad F2, zumindest einen weiteren Durchflusssensor S2 und zumindest ein weiteres Ventil V2. Der zumindest eine weitere Fluidflusspfad F2 ist entsprechend dem zweiten Fluidflusspfad F1 aufgebaut. Somit ist der zumindest eine weitere Fluidflusspfad F2 strömungsmechanisch parallel zu dem ersten Fluidflusspfad F0 und dem zweiten Fluidflusspfad F1 angeordnet. Der zumindest eine weitere Durchflusssensor S2 ist ausgebildet, um im Rahmen zumindest eines von dem ersten Messbereich und dem zweiten Messbereich unterschiedlichen, weiteren Messbereichs den Volumenstrom in dem zumindest einen weiteren Fluidflusspfad F2 zu erfassen und zumindest ein weiteres Sensorsignal y2 bereitzustellen, das weitere Messwerte des erfassten Volumenstroms in dem zumindest einen weiteren Fluidflusspfad F2 repräsentiert. Das zumindest eine weitere Ventil V2 ist zum Freigeben und Sperren des zumindest einen weiteren Fluidflusspfads F2 mittels eines weiteren Steuersignals x2 ansteuerbar.

Bei der Sensoreinheit 1 gemäß dem hier dargestellten Ausführungsbeispiel können beliebige Arten von Durchflussmessern bzw. Durchflusssensoren verwendet werden. Der Einfachheit halber kann angenommen werden, dass es sich bei den dargestellten Sensoren um kalorimetrisch wirkende Durchflusssensoren handelt. Innerhalb der Sensoreinheit 1 existieren mindestens zwei parallele Stränge bzw. Fluidflusspfade F0 und F1, durch welche das zu messende Medium strömen kann. Der erste Fluidflusspfad F0 umfasst einen inline verbauten Durchflusssensor S0. Dieser Fluidflusspfad F0 wird auch als Initialstrang (IS) bezeichnet und dient der Erfassung aller Messwerte im ersten Messbereich bzw. Messintervall.

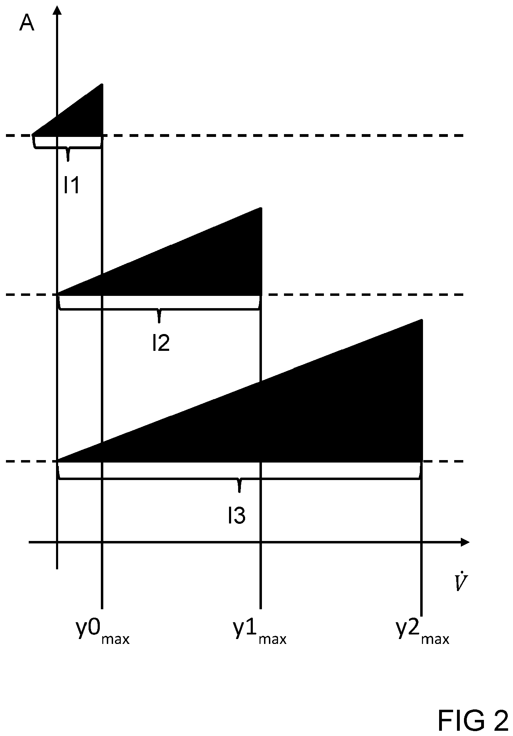

Figur 2 zeigt ein exemplarisches Volumenstrom-Amplitude-Diagramm gemäß einem Ausführungsbeispiel. Hierbei ist an einer Abszissenachse des Diagramms ein Volumenstrom $\dot{V}$ aufgetragen und ist an einer Ordinatenachse des Diagramms eine Amplitude A aufgetragen. Ferner sind Obergrenzen $y0_{max}$, $y1_{max}$ und $y2_{max}$ von Messbereichen I1, I2 und I3 aller drei Durchflusssensoren aus Figur 1 eingezeichnet.

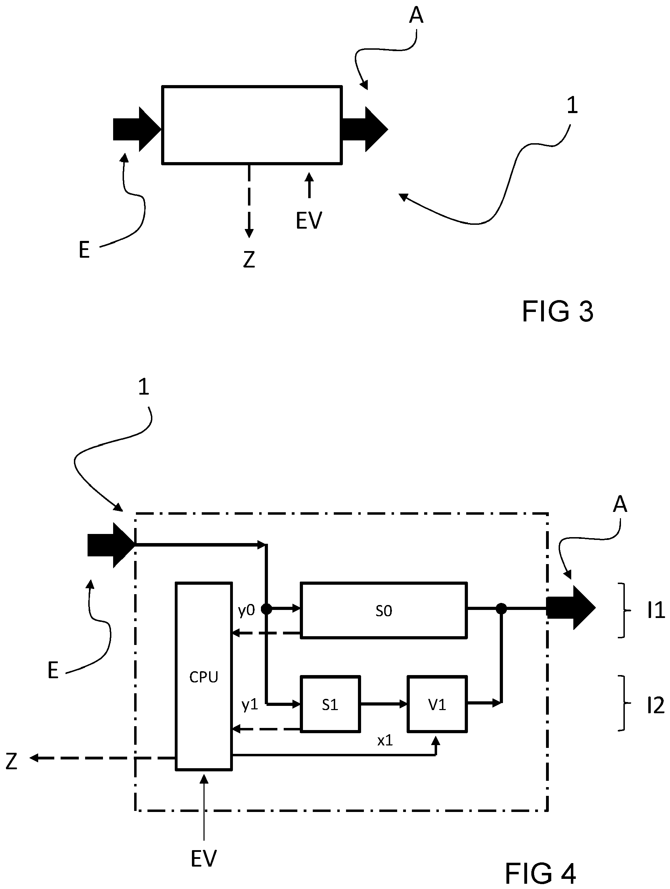

Figur 3 zeigt eine schematische Darstellung einer Sensoreinheit 1 gemäß einem Ausführungsbeispiel. Die Sensoreinheit 1 entspricht oder ähnelt hierbei der Sensoreinheit aus Figur 1. In der Darstellung von Figur 3 sind von der Sensoreinheit 1 hierbei lediglich der Eingangsanschluss E, der Ausgangsanschluss A, das Ausgangssignal Z und symbolisch eine Energieversorgung EV eingezeichnet. Anders ausgedrückt ist die Sensoreinheit 1 in Figur 3 in grober Abstraktion dargestellt. Es ist eine Sensoreinheit 1 gezeigt, die über einen Eingangsanschluss E und einen Ausgangsanschluss A verfügt. Zudem ist eine Energieversorgung EV gezeigt. Die Sensoreinheit 1 liefert ein digitales Ausgangssignal Z, das von einer Steuerung oder Geräte-CPU oder Anzeigeeinheit ausgelesen und angezeigt werden kann.

Figur 4 zeigt eine schematische Darstellung einer Sensoreinheit 1 gemäß einem Ausführungsbeispiel. Die Sensoreinheit 1 in Figur 4 entspricht hierbei der Sensoreinheit aus Figur 1 mit Ausnahme dessen, dass die Sensoreinheit 1 in Figur 4 lediglich den ersten Fluidflusspfad und den zweiten Fluidflusspfad aufweist, zusätzlich eine Energieversorgung EV symbolisch angedeutet ist und der erste Fluidflusspfad durch den ersten Messbereich I1 bezeichnet ist und der zweite Fluidflusspfad durch den zweiten Messbereich I2 bezeichnet ist.

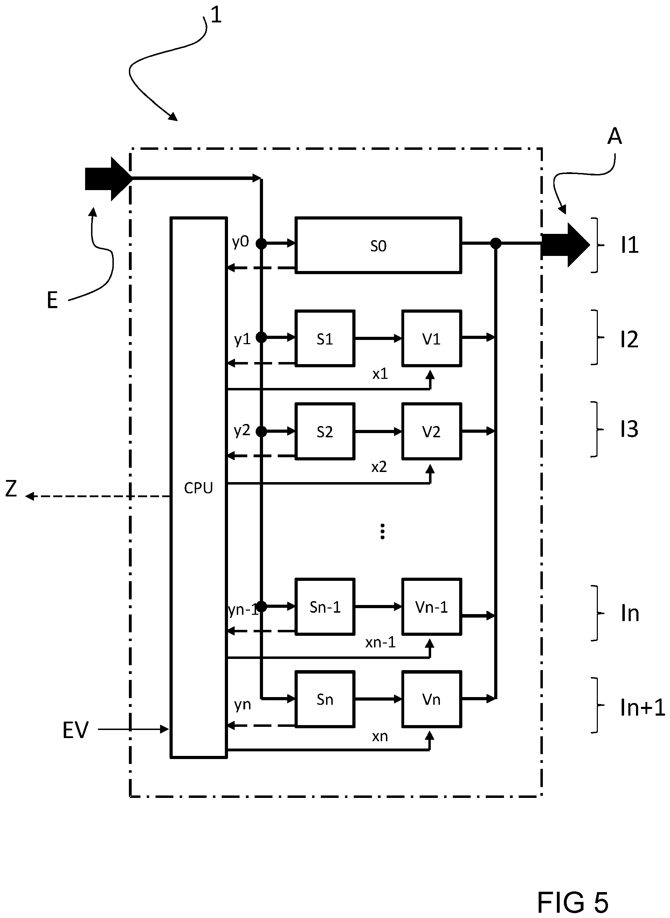

Figur 5 zeigt eine schematische Darstellung einer Sensoreinheit 1 gemäß einem Ausführungsbeispiel. Die Sensoreinheit 1 in Figur 5 entspricht hierbei der Sensoreinheit aus Figur 1 bzw. Figur 4 mit Ausnahme dessen, dass für die Sensoreinheit 1 in Figur 5 eine Mehrzahl von Fluidflusspfaden dargestellt ist, wobei die Fluidflusspfade jeweils durch ihre Messbereiche bezeichnet sind. Genauer gesagt sind in Figur 5 lediglich beispielhaft fünf Fluidflusspfade der Sensoreinheit 1 gezeigt, wobei mögliche zusätzliche Fluidflusspfade in der Darstellung symbolisch angedeutet sind. Zusätzlich zu den drei Fluidflusspfaden aus Figur 1 und deren Messbereichen I1, I2 und I3 sind mit möglichen, dazwischenliegenden, zusätzlichen Fluidflusspfaden auch ein vorletzter Fluidflusspfad mit einem vorletzten Messbereich In, einem vorletzten Durchflusssensor Sn-1, einem vorletzten Sensorsignal yn-1, einem vorletzten Ventil Vn-1 und einem vorletzten Steuersignal xn-1, und ein letzter Fluidflusspfad mit einem letzten Messbereich In+1, einem letzten Durchflusssensor Sn, einem letzten Sensorsignal yn, einem letzten Ventil Vn und einem letzten Steuersignal xn gezeigt. Zudem ist eine Energieversorgung EV für die Sensoreinheit und somit für die Steuereinheit CPU eingezeichnet.

Anders ausgedrückt ist mindestens ein weiterer zum ersten Fluidflusspfad bzw. Initialstrang parallel verlaufender Fluidflusspfad für die Sensoreinheit 1 notwendig. Alle parallel angeordneten Fluidflusspfade bzw. Stränge werden nachfolgend auch als Erweiterungsstränge bezeichnet, die weitere Messbereiche bzw. Messintervalle I2 bis In+1 des gesamten Messbereichs der Sensoreinheit 1 abdecken. Jeder Erweiterungsstrang ist strukturell gleich aufgebaut. Jeder Erweiterungsstrang umfasst einen Durchflusssensor S1 bis Sn sowie ein nachfolgend, sequentiell verbautes 2/2-Wege-Ventil V1 bis Vn. Die Ventile V1 bis Vn sind im stromlosen Zustand geschlossen, wodurch im stromlosen Zustand nur der Initialstrang durchströmt werden kann.

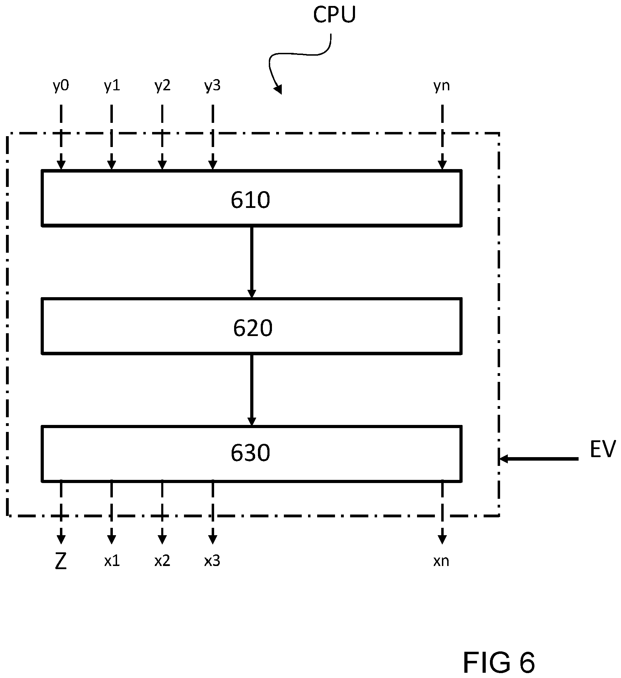

Figur 6 zeigt eine schematische Darstellung einer Steuereinheit CPU gemäß einem Ausführungsbeispiel. Die Steuereinheit CPU entspricht hierbei der Steuereinheit der Sensoreinheit aus Figur 5. Bezüglich der Steuereinheit CPU sind in der Darstellung von Figur 6 hierbei eine Mehrzahl von Sensorsignalen y0 bis yn, eine Mehrzahl von Steuersignalen x1 bis xn, das Ausgangssignal Z und die Energieversorgung EV gezeigt. Ferner ist gezeigt, dass die Steuereinheit CPU einen ersten Signalwandler 610 bzw. eine Einleseeinrichtung, eine Informationsverarbeitungseinrichtung 620 bzw. Verarbeitungseinrichtung und einen zweiten Signalwandler 630 bzw. eine Bereitstellungseinrichtung aufweist. Der erste Signalwandler 610 ist ausgebildet, um die Sensorsignale y0 bis yn einzulesen und der Informationsverarbeitungseinrichtung 620 zur Verfügung zu stellen. Der zweite Signalwandler 630 ist ausgebildet, um Steuersignale x1 bis xn und das Ausgangssignal Z bereitzustellen.

Anders ausgedrückt umfasst die Sensoreinheit die Steuereinheit CPU, welche das Durchfluss-Ausgangssignal bzw. Ausgangssignal Z bereitstellt oder ausgibt. Darüber hinaus werden von der Steuereinheit CPU die Steuersignale x1 bis xn ausgegeben, welche die in der Sensoreinheit verbauten Ventile V1 bis Vn umschalten lassen können. Die Steuereinheit CPU umfasst weitere Eingänge, über welche die einzelnen Sensorsignale y0 bis yn der in der Sensoreinheit verbauten Durchflusssensoren S0 bis Sn eingelesen werden, die mittels der Informationsverarbeitungseinrichtung 620 zum Ausgabesignal Z umgerechnet werden.

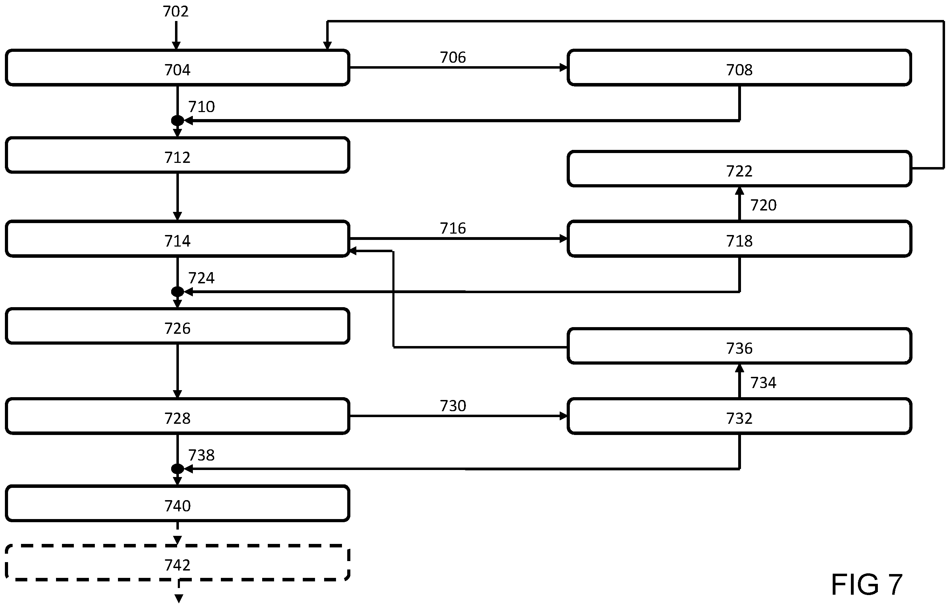

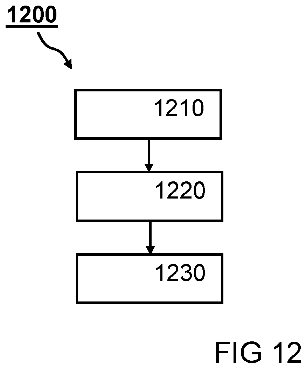

Figur 7 zeigt ein Ablaufdiagramm eines Messverfahrens gemäß einem Ausführungsbeispiel. Das in Figur gezeigte Messverfahren wird im Zusammenhang mit der Sensoreinheit aus einer der vorstehend beschriebenen Figuren insbesondere mittels der Verarbeitungseinrichtung bzw. Informationsverarbeitungseinrichtung der Steuereinheit aus Figur 6 und/oder im Rahmen des Verarbeitungsschrittes des Steuerverfahrens aus Figur 12 durchgeführt.

Bei 702 erfolgt ein Einschalten der Energieversorgung für die Steuereinheit. Bei 704 wird der erste Messbereich I1 gemessen. Wenn wie bei 706 das erste Sensorsignal y0 des ersten Durchflusssensors S0 kleiner als der obere Grenzwert $y0_{max}$ des ersten Messbereichs I1 ist, erfolgt bei 708 eine Ausgabe des Ausgangssignals Z mit Z = f(y0). Wenn wie bei 710 das das erste Sensorsignal y0 des ersten Durchflusssensors S0 größer oder gleich dem oberen Grenzwert $y0_{max}$ des ersten Messbereichs I1 ist, so wird bei 712 das erste Ventil V1 eingeschaltet.

Ausgehend von 712 wird bei 714 nachfolgend der zweite Messbereich I2 gemessen. Wenn wie bei 716 das zweite Sensorsignal y1 des zweiten Durchflusssensors S1 kleiner als der obere Grenzwert $y1_{max}$ des zwei-

ten Messbereichs I2 ist, erfolgt bei 718 eine Ausgabe des Ausgangssignals Z mit Z = f(y0,y1). Wenn ausgehend von 718 wie bei 720 das zweite Sensorsignal y1 des zweiten Durchflusssensors S1 kleiner als 10 Prozent des oberen Grenzwerts $y1_{max}$ des zweiten Messbereichs I2 ist, wird bei 722 das erste Ventil V1 ausgeschaltet und geht das Messverfahren zurück zu 704. Wenn jedoch wie bei 724 das zweite Sensorsignal y1 des zweiten Durchflusssensors S1 größer oder gleich dem oberen Grenzwert $y1_{max}$ des zweiten Messbereichs I2 ist, werden bei 726 das zumindest eine weitere Ventil bzw. zweite Ventil V2 eingeschaltet und das erste Ventil V1 ausgeschaltet.

Ausgehend von 726 wird bei 728 der zumindest eine weitere Messbereich bzw. dritte Messbereich I3 gemessen. Wenn wie bei 730 das zumindest eine weitere Sensorsignal bzw. dritte Sensorsignal y2 des dritten Durchflusssensors S2 kleiner als der obere Grenzwert $y2_{max}$ des dritten Messbereichs I3 ist, erfolgt bei 732 eine Ausgabe des Ausgangssignals Z mit Z = f(y0,y2). Wenn ausgehend von 732 wie bei 734 das dritte Sensorsignal y2 des dritten Durchflusssensors S2 kleiner als 10 Prozent des oberen Grenzwerts $y2_{max}$ des dritten Messbereichs I3 ist, wird bei 736 das dritte Ventil V2 ausgeschaltet und geht das Messverfahren zurück zu 714. Wenn jedoch wie bei 738 das dritte Sensorsignal y2 des dritten Durchflusssensors S2 größer oder gleich dem oberen Grenzwert $y2_{max}$ des dritten Messbereichs I3 ist, so wird bei 740 das dritte Ventil V3 eingeschaltet und wird das zweite Ventil V2 ausgeschaltet.

Ferner ist in Figur 7 angedeutet, dass ausgehend von 740 nachfolgend bei 742 der dritte Messbereich I3 gemessen wird.

Anders ausgedrückt und unter Bezugnahme auf die vorstehend beschriebenen Figuren startet das in Figur 7 gezeigte Messverfahren, sobald die Energieversorgung (EV) der Sensoreinheit eingeschaltet wird. Der Vorgang beginnt in diesem Ausführungsbeispiel stets mit dem kleinstmöglichen Durchfluss des empfindlichsten Fluidflusspfads bzw. Strangs, dem Initialstrang (eine reziproke Vorgehensweise beginnend mit dem größtmöglichen Durchfluss ist ebenfalls möglich). Alle Ventile V1 bis Vn sperren. Das erste Sensorsignal y0 des ersten Strangs bzw. Fluidflusspfads wird geprüft. Ist y0 kleiner als der maximale Messbereich des ersten Durchflusssensors S0, wird über eine Funktion f(y0) der Hauptausgabewert bzw. das Ausgangssignal Z ausgegeben. Wird der Messbereich von y0 überschritten, d.h. der erste Durchflusssensor S0 gibt den Wert $y0_{max}$ aus, wird das Ventil, hier das erste Ventil V1, des nachfolgenden Strangs geschaltet. Das zweite Sensorsignal y1 des zweiten Strangs bzw. Fluidflusspfads wird geprüft. Ist y1 kleiner als der maximale Messbereich des zweiten Durchflusssensors S1, wird über eine Funktion f(y0,y1) der Hauptausgabewert bzw. das Ausgangssignal Z ausgegeben. Bewegt sich im Laufe der Messung der Sensorwert des zweiten Sensorsignals y1 des zweiten Durchflusssensors S1 in den unteren Messbereich, d.h.

das zweite Ausgangssignal y1 ist kleiner als 10 Prozent des oberen Grenzwerts des zweiten Messbereichs, wird das erste Ventil V1 geschlossen und das Messverfahren springt zurück zu dem vorherigen Messintervall, in diesem Fall den Initialstrang. Wird der Messbereich von y1 bzw. S1 überschritten, d.h. der zweite Durchflusssensor S1 gibt den Wert $y1_{max}$ aus, wird das Ventil des nachfolgenden Strangs bzw. Fluidflusspfads geschaltet. Das dritte Sensorsignal y2 des dritten Strangs wird geprüft. Ist y2 kleiner als der maximale Messbereich des dritten Durchflusssensors S2, wird über eine Funktion f(y0,y2) der Hauptausgabewert bzw. das Ausgangssignal Z ausgegeben. Bewegt sich im Laufe der Messung der Sensorwert des dritten Sensorsignals y2 von des dritten Durchflusssensors S2 in den unteren Messbereich, d.h. das dritte Ausgangssignal y2 ist kleiner als 10 Prozent des oberen Grenzwerts des dritten Messbereichs, wird das dritte Ventil V2 geschlossen und das Messverfahren springt zurück zu dem vorherigen Messintervall bzw. Messbereich, in diesem Fall I2. Dieses Verhalten erfolgt für alle Stränge bzw. Fluidflusspfade ähnlich bis zum letzten Messbereich In+1. der letzte Messbereich In+1. ist so auszulegen, dass für den jeweiligen Anwendungsfall kein Überschreiten von dessen oberen Grenzwert $yn_{max}$ erfolgt.

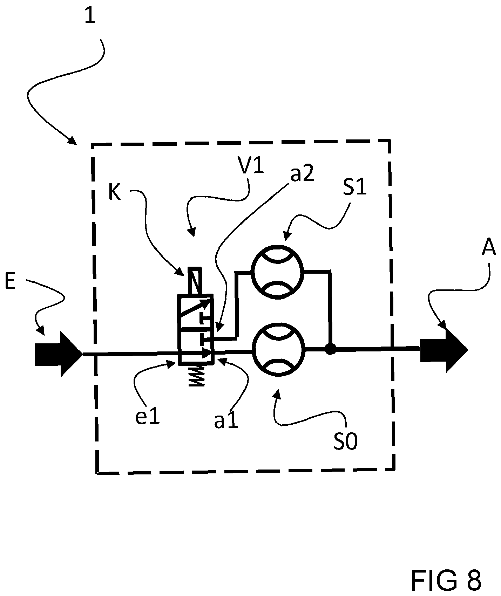

Figur 8 zeigt eine schematische Darstellung einer Sensoreinheit 1 gemäß einem Ausführungsbeispiel. Die Sensoreinheit 1 in Figur 8 entspricht der Sensoreinheit aus Figur 1 bzw. Figur 4 mit Ausnahme dessen, dass das zumindest eine Ventil V1 zwischen dem Eingangsanschluss E und einer Verzweigungsstelle des Fluidkanals in den ersten Fluidflusspfad und den zweiten Fluidflusspfad angeordnet ist, in Fluidflussrichtung stromaufwärts der Durchflusssensoren S0 und S1 angeordnet ist und als ein 3/2-Wege-Ventil ausgeführt ist.

Die Sensoreinheit 1 umfasst den Eingangsanschluss E und den Ausgangsanschluss A. Im Inneren der Sensoreinheit 1 ist ein federrückgestelltes (oder pneumatisch rückgestelltes) Magnetventil V1 samt elektromechanischer Spule K verbaut. Das Ventil V1 weist zwei Schaltstellungen auf und umfasst über drei Anschlüsse. Ein Eingang e1 des Ventils V1 ist mit dem Eingangsanschluss E verbunden. Ein im stromlosen Zustand aktiver Ausgang a1 des Ventils V1 ist mit dem ersten Durchflusssensor S0 verbunden, der mit dem Ausgangsanschluss A der Sensoreinheit 1 verbunden ist. Ein im geschalteten Zustand aktiver Ausgang a2 des Ventils V1 ist mit dem zweiten Durchflusssensor S1 verbunden, der ebenfalls mit dem Ausgangsanschluss A der Sensoreinheit 1 verbunden ist. Wird die Spule K des Ventils V1 geschaltet, kann zwischen den beiden Durchflusssensoren S0 und S1 umgeschaltet werden.

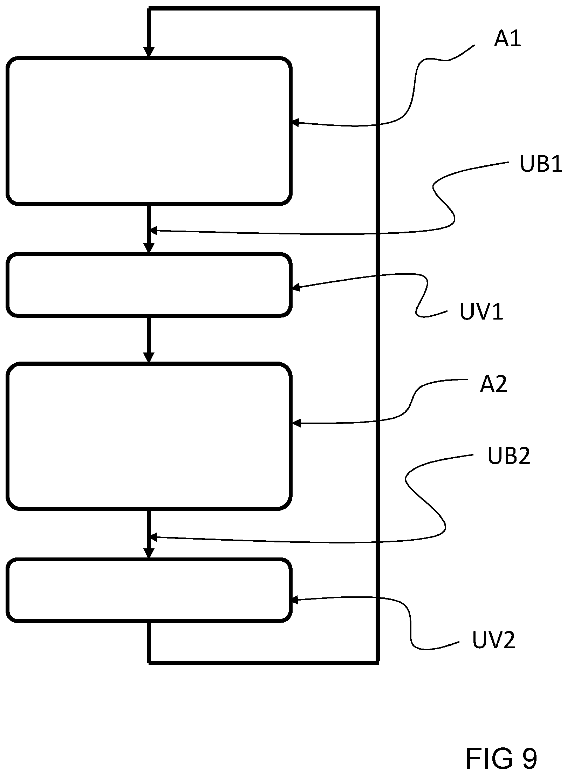

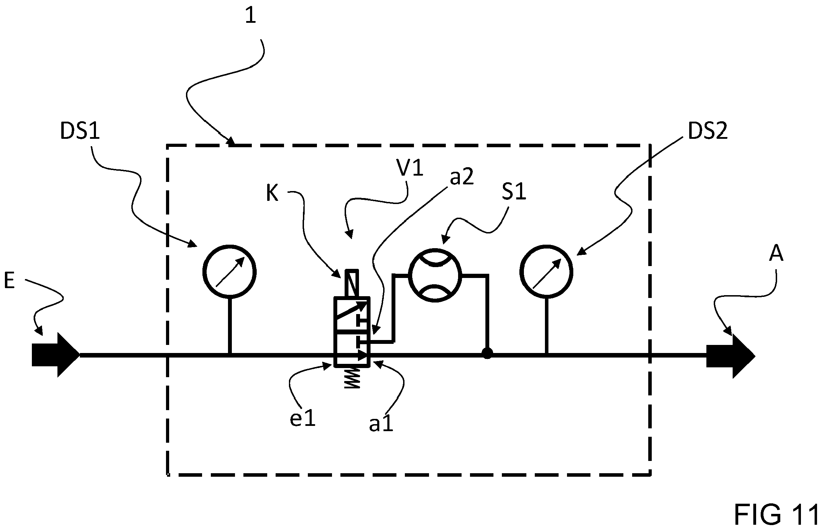

Figur 9 zeigt ein Ablaufdiagramm eines Messverfahrens gemäß einem Ausführungsbeispiel. Das in Figur gezeigte Messverfahren wird im Zusammenhang mit der Sensoreinheit aus Figur 8 oder 11 insbesondere mittels der Verarbeitungseinrichtung bzw. Informationsverarbeitungseinrichtung der Steuereinheit aus Figur 6 und/oder im Rahmen des Verarbeitungsschrittes des Steuerverfahrens aus Figur 12 durchgeführt. Das Messverfahren dient zum Messen des Volumenstroms.

Das Messverfahren läuft in einer Schleife ab. Es existieren insgesamt vier verschiedene Zustände bzw. Aktionen. Während des ersten, initialen Zustandes A1 wird der Ausgabewert Beziehung weise das Ausgangssignal Z berechnet und ausgegeben. Z berechnet sich aus dem Mittelwert der letzten n Messwerte des ersten Sensorsignals y0, genauer gesagt $Z = \frac{1}{i} \sum_{i=1}^{n} y_{0,i}$. Während des Zustandes A1 wird eine Messung mit großem zulässigen Wertebereich durchgeführt. Wird während der Messung irgendwann eine erste Umschaltbedingung UB1 erfüllt, wobei UB1 als $Z \leq m \cdot y0_{max}$ definiert ist, wird das Ventil V1 durch Einschalten der Magnetspule K eingeschaltet, was als UV1 eingezeichnet ist. Die erste Umschaltbedingung UB1 prüft, ob das Ausgangssignal Z zu einer Zeit während der Messung so klein wird, dass in einen empfindlicheren Messzustand A2 gewechselt wird. Der Faktor m gibt an, bei welchem Grenzwert, prozentual vom oberen Grenzwert, also dem maximalen Messbereich des ersten Durchflusssensors S0, ein Messzustandswechsel erfolgen soll. Ein typischer Wert für m ist beispielsweise 0,1. Während des empfindlicheren Messzustands A2 wird das Ausgangssignal Z aus dem Mittelwert der letzten n Messwerten des zweiten Sensorsignals y1 berechnet, genauer gesagt gemäß $Z = \frac{1}{i} \sum_{i=1}^{n} y_{1,i}$. Während des empfindlicheren Messzustands A2 wird eine Messung mit kleinerem zulässigen Messbereich, aber höherer Empfindlichkeit durchgeführt. Wird während der Messung irgendwann eine zweite Umschaltbedingung UB2 erfüllt, wobei UB2 als $Z \geq r \cdot y1_{max}$ definiert ist, wird das Ventil V1 durch Ausschalten der Magnetspule K ausgeschaltet, was als UV2 eingezeichnet ist. Die zweite Umschaltbedingung UB2 prüft, ob das Ausgangssignal Z zu einer Zeit während der Messung so groß wird, dass in den größeren Messbereich, also den initialen Zustand A1 gewechselt wird. Der Faktor r gibt an, bei welchem Grenzwert, prozentual vom oberen Grenzwert, also dem maximalen Messbereich des zweiten Durchflusssensors S1, ein Messzustandswechsel erfolgen soll. Ein typischer Wert für r ist beispielsweise 0,9. Diese Wechsel der Messzustände, hier von A1 nach A2 und von A2 nach A1, sind von der Messgröße abhängig und erfolgen automatisch durch die hinterlegte Logik der Steuereinheit.

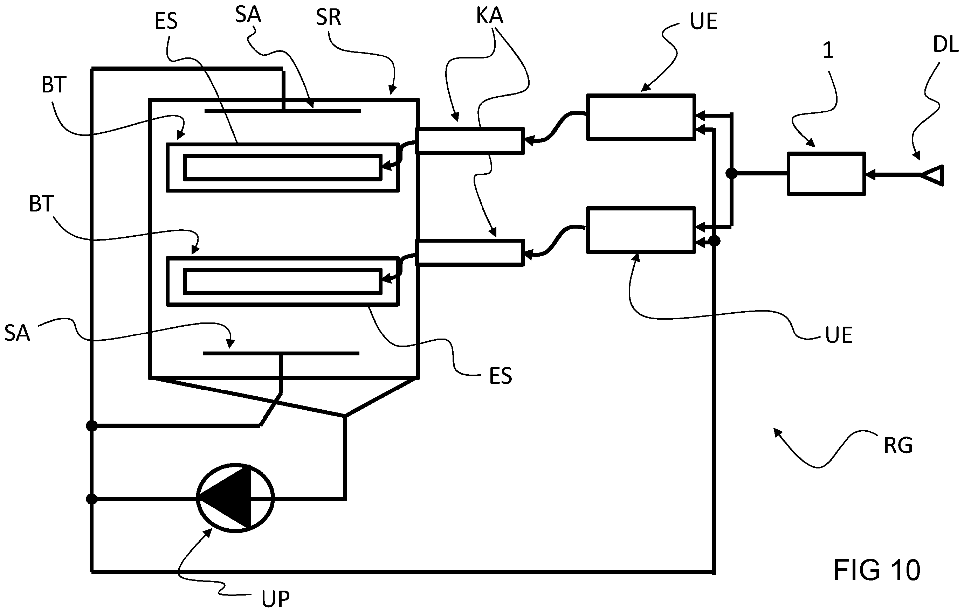

Figur 10 zeigt eine schematische Darstellung eines Reinigungsgeräts RG gemäß einem Ausführungsbeispiel. Bei dem Reinigungsgerät RG handelt es sich um ein Reinigungsgerät für medizinisches Reinigungsgut, insbesondere zumindest ein Endoskop ES. Das Reinigungsgerät RG umfasst einen Spülraum SR zum Aufnehmen des Reinigungsguts, hier zumindest eines Endoskops ES, wobei der Spülraum zumindest eine Fluidschnittstelle KA zur strömungsmechanischen Kopplung mit dem Reinigungsgut aufweist. Die zumindest eine Fluidschnittstelle KA ist jeweils als eine Korbankopplung ausgeführt. Das Reinigungsgerät RG umfasst ferner eine Bereitstellungseinrichtung DL zum Bereitstellen von Druckluft. Die Bereitstellungseinrichtung DL ist strömungsmechanisch mit der zumindest einen Fluidschnittstelle KA verbunden. Auch umfasst das Reinigungsgerät RG eine Sensoreinheit 1, die der Sensoreinheit aus einer der hierin beschriebenen Figuren entspricht oder ähnelt. Die Sensoreinheit 1 ist strömungsmechanisch zwischen die Bereitstellungseinrichtung DL und die zumindest eine Fluidschnittstelle KA geschaltet.

Somit wird die Sensoreinheit 1 in dem Reinigungsgerät RG im Rahmen eines Reinigungs-und Desinfektionsprozesses von insbesondere englumigem, medizinischen Spülgut bzw. Reinigungsgut, hier zumindest eines Endoskops ES, insbesondere für Messaufgaben im Hinblick auf eine Drucklufteinspeisung beim Reinigungsschritt oder Trocknungsschritt verwendet. Somit kommt die Sensoreinheit 1 bei einer Anwendung zum Einsatz, die bei der Durchflussmessung von Endoskopen ES vorkommt, und somit in einem Reinigungsgerät RG, welches die Aufgabe der Endoskopaufbereitung oder Endoskopüberprüfung hat.

Gemäß dem in Figur 10 dargestellten Ausführungsbeispiel umfasst das Reinigungsgerät RG ferner zumindest eine Überwachungseinheit UE, die zwischen die Sensoreinheit 1 und die zumindest eine Fluidschnittstelle KA geschaltet ist, eine Umwälzpumpe UP zum Fördern von Fluid in den und aus dem Spülraum SR sowie in dem Spülraum SR zumindest einen Sprüharm SA und zumindest einen Beladungsträger BT. In der Darstellung von Figur 10 sind beispielhaft lediglich zwei Endoskope ES in jeweils einem eigenen Beladungsträger BT in dem Spülraum SR angeordnet. Jeder Beladungsträger BT und damit jedes Endoskop ES ist mit einer eigenen Fluidschnittstelle KA bzw. einer eigenen Korbankopplung gekoppelt. Jede Fluidschnittstelle KA ist über eine eigene Überwachungseinheit UE über die Sensoreinheit 1 mit der Bereitstellungseinrichtung DL verbunden.

Figur 11 zeigt eine schematische Darstellung einer Sensoreinheit 1 gemäß einem Ausführungsbeispiel. Die Sensoreinheit 1 in Figur 11 entspricht der Sensoreinheit aus Figur 8 mit Ausnahme dessen, dass der erste Durchflusssensor einen Differenzdrucksensor mit einem ersten Differenzdruck-Sensorelement DS1 und einem zweiten Differenzdruck-Sensorelement DS2 aufweist. Das Ventil V1 und der zweite Fluidflusspfad mit dem zweiten Durchflusssensor S1 sind hierbei zwischen den Differenzdrucksensoren DS1 und DS2 angeordnet.

Figur 12 zeigt ein Ablaufdiagramm eines Verfahrens 1200 zum Steuern gemäß einem Ausführungsbeispiel. Das Verfahren 1200 zum Steuern ist ausführbar, um die Sensoreinheit aus einer der vorstehend beschriebenen Figuren oder eine ähnliche Sensoreinheit zu steuern. Hierbei ist das Verfahren 1200 mittels bzw.

unter Verwendung der Steuereinheit aus einer der vorstehend beschriebenen Figuren oder einer ähnlichen Steuereinheit ausführbar. Das Verfahren 1200 zum Steuern umfasst einen Schritt 1210 des Einlesens, einen Schritt 1220 des Verarbeitens und einen Schritt 1230 des Bereitstellens.

Im Schritt 1210 des Einlesens wird zumindest eines der Sensorsignale von zumindest einem der Durchflusssensoren der Sensoreinheit eingelesen. Nachfolgend wird im Schritt 1220 des Verarbeitens das zumindest eine Sensorsignal verarbeitet, um den Volumenstrom zu bestimmen, das Ausgangssignal zu erzeugen und das Steuersignal zu generieren, und werden im Schritt 1230 des Bereitstellens das Steuersignal zur Ausgabe an das zumindest eine Ventil bereitgestellt und das Ausgangssignal bereitgestellt. Im Zusammenhang mit dem Verfahren 1200 zum Steuern sei auch auf Figur 7 und Figur 9 verwiesen.

## Patentansprüche

1. Sensoreinheit (1) zum Bestimmen eines Volumenstroms eines Fluides für einen Reinigungsvorgang von medizinischem Reinigungsgut (ES), wobei die Sensoreinheit (1) folgende Merkmale aufweist:

   einen Eingangsanschluss (E) zum Einleiten des Fluides in die Sensoreinheit (1), einen Ausgangsanschluss (A) zum Ausleiten des Fluides aus der Sensoreinheit (1) und einen Fluidkanal mit einem ersten Fluidflusspfad (F0) und mit einem zu dem ersten Fluidflusspfad (F0) strömungsmechanisch parallelen, zweiten Fluidflusspfad (F1), wobei der Eingangsanschluss (E) und der Ausgangsanschluss (A) über den Fluidkanal strömungsmechanisch miteinander verbunden sind;
   einen ersten Durchflusssensor (S0) mit einem ersten Messbereich (I1) und einen zweiten Durchflusssensor (S1) mit einem von dem ersten Messbereich (I1) unterschiedlichen, zweiten Messbereich (I2), wobei der erste Durchflusssensor (S0) ausgebildet ist, um im Rahmen des ersten Messbereichs (I1) den Volumenstrom in dem ersten Fluidflusspfad (F0) zu erfassen und ein erstes Sensorsignal (y0) bereitzustellen, das erste Messwerte des erfassten Volumenstroms in dem ersten Fluidflusspfad (F0) repräsentiert, wobei der zweite Durchflusssensor (S1) ausgebildet ist, um im Rahmen des zweiten Messbereichs (I2) den Volumenstrom in dem zweiten Fluidflusspfad (F1) zu erfassen und ein zweites Sensorsignal (y1) bereitzustellen, das zweite Messwerte des erfassten Volumenstroms in dem zweiten Fluidflusspfad (F1) repräsentiert; zumindest ein Ventil (V1), das zum Freigeben und Sperren zumindest eines der Fluidflusspfade (F0, F1) für einen Durchfluss des Fluides mittels eines Steuersignals (x1) ansteuerbar ist; und
   eine Steuereinheit (CPU), die ausgebildet ist, um abhängig von zumindest einem der Sensorsignale (y0, y1) das Steuersignal (x1) bereitzustellen, und abhängig von zumindest einem der Sensorsignale (y0, y1) den Volumenstrom des Fluids zu bestimmen und ein den Volumenstrom des Fluids repräsentierendes Ausgangssignal (Z) bereitzustellen.

2. Sensoreinheit (1) gemäß Anspruch 1, wobei die Durchflusssensoren (S0, S1) Volumenstromsensoren und/oder Massenstromsensoren aufweisen, und/oder wobei zumindest einer der Durchflusssensoren (S0, S1) in dem ersten Fluidflusspfad (F0) oder in dem zweiten Fluidflusspfad (F1) angeordnet ist, und/oder wobei zumindest einer der Durchflusssensoren (S0, S1) einen Flügelradzähler, einen Ovalradzähler, einen Differenzdrucksensor oder einen kalorimetrisch wirkenden Durchflusssensor aufweist.

3. Sensoreinheit (1) gemäß einem der vorangegangenen Ansprüche, wobei der erste Messbereich (I1) kleiner als der zweite Messbereich (I2) ist, und/oder wobei sich die Messbereiche (I1, I2) zumindest teilweise überlappen, und/oder wobei die Steuereinheit (CPU) ausgebildet ist, um das zumindest eine Ventil (V1) anzusteuern, um den zweiten Fluidflusspfad (F1) zu sperren, wenn die ersten Messwerte unterhalb eines für den ersten Messbereich (I1) gültigen, ersten oberen Schwellenwertes liegen oder die zweiten Messwerte unterhalb eines für den zweiten Messbereich (I2) gültigen, zweiten unteren Schwellenwertes liegen.

4. Sensoreinheit (1) gemäß einem der Ansprüche 1 bis 2, wobei der erste Messbereich (I1) größer als der zweite Messbereich (I2) ist, und/oder wobei sich die Messbereiche (I1, I2) zumindest teilweise überlappen, und/oder wobei die Steuereinheit (CPU) ausgebildet ist, um das zumindest eine Ventil (V1) anzusteuern, um den zweiten Fluidflusspfad (F1) zu sperren, wenn der durch das Ausgangssignal (Z) repräsentierte Volumenstrom oberhalb eines für den ersten Messbereich (I1) gültigen, ersten unteren Schwellenwertes oder oberhalb eines für den zweiten Messbereich (I2) gültigen, zweiten oberen Schwellenwertes liegt.

5. Sensoreinheit (1) gemäß einem der vorangegangenen Ansprüche, wobei das zumindest eine Ventil (V1) in dem zweiten Fluidflusspfad (F1) angeordnet ist, und/oder wobei das zumindest eine Ventil (V1) in Fluidflussrichtung stromabwärts des zweiten Durchflusssensors (S1) angeordnet ist, und/oder wobei das zumindest eine Ventil (V1) als ein normalerweise geschlossenes Ventil oder normalerweise geschlossenes 2/2-Wege-Ventil ausgeführt ist.

6. Sensoreinheit (1) gemäß einem der Ansprüche 1 bis 4, wobei das zumindest eine Ventil (V1) zwischen dem Eingangsanschluss (E) und einer Verzweigungsstelle des Fluidkanals in den ersten Fluidflusspfad (F0) und den zweiten Fluidflusspfad (F1) angeordnet ist, und/oder wobei das zumindest eine Ventil (V1) in Fluidflussrichtung stromaufwärts der Durchflusssensoren (S0, S1) angeordnet ist, und/oder wobei das zumindest eine Ventil (V1) als ein 3/2-Wege-Ventil ausgeführt ist.

7. Sensoreinheit (1) gemäß Anspruch 6, wobei der erste Durchflusssensor (S0) einen Differenzdrucksensor (DS1, DS2) aufweist und der zweite Durchflusssensor (S1) einen in dem zweiten Fluidflusspfad (F1) angeordneten Volumenstromsensor aufweist.

8. Sensoreinheit (1) gemäß einem der vorangegangenen Ansprüche, wobei die Steuereinheit (CPU) ausgebildet ist, um den Volumenstrom durch Mittelwertbildung unter Verwendung einer Mehrzahl von Messwerten zu bestimmen.

9. Sensoreinheit (1) gemäß einem der vorangegangenen Ansprüche, mit zumindest einem weiteren Fluidflusspfad, zumindest einem weiteren Durchflusssensor (S2, Sn-1, Sn) und zumindest einem weiteren Ventil (V2, Vn-1, Vn), wobei der zumindest eine weitere Fluidflusspfad strömungsmechanisch parallel zu dem ersten Fluidflusspfad (F0) und dem zweiten Fluidflusspfad (F1) angeordnet ist, wobei der zumindest eine weitere Durchflusssensor (S2, Sn-1, Sn) ausgebildet ist, um im Rahmen zumindest eines von dem ersten Messbereich (I1) und dem zweiten Messbereich (I2) unterschiedlichen, weiteren Messbereichs (I3, In, In+1) den Volumenstrom in dem zumindest einen weiteren Fluidflusspfad zu erfassen und zumindest ein weiteres Sensorsignal (y2, yn-1, yn) bereitzustellen, das weitere Messwerte des erfassten Volumenstroms in dem zumindest einen weiteren Fluidflusspfad repräsentiert, wobei das zumindest eine weitere Ventil (V2, Vn-1, Vn) zum Freigeben und Sperren des zumindest einen weiteren Fluidflusspfads ansteuerbar ist.

10. Reinigungsgerät (RG) für medizinisches Reinigungsgut (ES), wobei das Reinigungsgerät (RG) folgende Merkmale aufweist:

    einen Spülraum (SR) zum Aufnehmen des Reinigungsguts (ES), wobei der Spülraum (SR) zumindest eine Fluidschnittstelle (KA) zur strömungsmechanischen Kopplung mit dem Reinigungsgut (ES) aufweist;

eine Bereitstellungseinrichtung (DL) zum Bereitstellen von Druckluft, wobei die Bereitstellungseinrichtung (DL) strömungsmechanisch mit der zumindest einen Fluidschnittstelle (KA) verbunden ist; und

eine Sensoreinheit (1) gemäß einem der vorangegangenen Ansprüche, wobei die Sensoreinheit (1) strömungsmechanisch zwischen die Bereitstellungseinrichtung (DL) und die zumindest eine Fluidschnittstelle (KA) geschaltet ist.

11. Reinigungsgerät (RG) gemäß Anspruch 10, bei dem die zumindest eine Fluidschnittstelle (KA) zum Koppeln des Reinigungsguts (ES) in Form von englumigen, medizinischen Spülgütern, insbesondere von Endoskopen ausgeformt ist.

12. Reinigungsgerät (RG) gemäß Anspruch 10 oder 11, bei der die Bereitstellungseinrichtung (DL) zum Bereitstellen der Druckluft zur Drucklufteinspeisung zum Reinigen oder Trocknen des Reinigungsguts (ES) ausgebildet ist.

13. Verfahren (1200) zum Steuern einer Sensoreinheit (1) gemäß einem der Ansprüche 1 bis 9, wobei das Verfahren (1200) folgende Schritte aufweist:

    Einlesen (1210) zumindest eines der Sensorsignale (y0, y1, y2, yn-1, yn) von zumindest einem der Durchflusssensoren (S0, S1, S2, Sn-1, Sn); Verarbeiten (1220) des zumindest einen Sensorsignals (y0, y1, y2, yn-1, yn), um den Volumenstrom zu bestimmen, das Ausgangssignal (Z) zu erzeugen und das Steuersignal (x1, x2, xn-1, xn) zu generieren; und Bereitstellen (1230) des Steuersignals (x1, x2, xn-1, xn) zur Ausgabe an das zumindest eine Ventil (V1, V2, Vn-1, Vn) und des Ausgangssignals (Z).

14. Computer-Programmprodukt mit Programmcode zur Durchführung des Verfahrens (1200) gemäß Anspruch 13, wenn das Computer-Programmprodukt auf einer Vorrichtung oder Steuereinheit ausgeführt wird.

FIG 1

FIG 2

FIG 3

FIG 4

1

A

E

Z

CPU

EV

y0 S0 I1

y1 S1 V1 I2
x1

y2 S2 V2 I3
x2

⋮

yn-1 Sn-1 Vn-1 In
xn-1

yn Sn Vn In+1
xn

FIG 5

CPU

y0    y1    y2    y3                                    yn

| 610 |

| 620 |

| 630 |                                               EV

Z     x1    x2    x3                                    xn

FIG 6

FIG 7

FIG 8

FIG 9

FIG 10

FIG 11

EP 3 943 894 A1

**1200**

| 1210 |
|---|

| 1220 |
|---|

| 1230 |
|---|

FIG 12

**Europäisches Patentamt**
**European Patent Office**
**Office européen des brevets**

# EUROPÄISCHER RECHERCHENBERICHT

Nummer der Anmeldung

EP 21 18 6368

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br>Y | US 2018/024026 A1 (PROULX STEPHEN [US] ET AL) 25. Januar 2018 (2018-01-25)<br>* Absätze [0028], [0030], [0042], [0043], [0034]; Abbildung 1 *<br>----- | 1-4,6-8,<br>13,14<br>10-12 | INV.<br>G01F7/00<br>A61B1/12<br>A61B90/70 |
| X<br>A | US 2020/225070 A1 (HALIMI HENRY M [US] ET AL) 16. Juli 2020 (2020-07-16)<br>* Absätze [0013], [0023] - [0033]; Abbildungen 1,4 *<br>----- | 1,5,13,<br>14<br>7 | ADD.<br>G01F1/06<br>G01F1/10<br>G01F1/34<br>G01F1/68<br>G01F3/10 |
| X | US 2016/138351 A1 (DILLARD WALTER S [US] ET AL) 19. Mai 2016 (2016-05-19)<br>* Absätze [0094], [0095]; Abbildung 5 *<br>----- | 1,9,13,<br>14 | |
| X | US 2012/085434 A1 (POWANDA WILLIAM [US]) 12. April 2012 (2012-04-12)<br>* Absätze [0032], [0039], [0043]; Abbildung 3 *<br>----- | 1,13,14 | |
| X | DE 196 15 857 C1 (EHRLER ALOIS [DE]) 2. Oktober 1997 (1997-10-02)<br>* Spalte 3, Zeile 62 - Spalte 4, Zeile 35; Abbildung 1 *<br>----- | 1,13,14 | RECHERCHIERTE SACHGEBIETE (IPC) |
| X<br>A | US 2009/205400 A1 (MCPEAK LUTHER DONALD [US]) 20. August 2009 (2009-08-20)<br>* Absätze [0028] - [0037]; Abbildung 3 *<br>----- | 1,13,14<br>8 | G01F<br>A61B |
| X | US 5 088 322 A (FITZPATRICK JAMES [US] ET AL) 18. Februar 1992 (1992-02-18)<br>* Spalte 6, Zeile 38 - Spalte 7, Zeile 51 *<br>----- | 1,13,14 | |
| Y | US 5 425 815 A (PARKER GEORGE C [GB] ET AL) 20. Juni 1995 (1995-06-20)<br>* Spalte 2, Zeile 31 - Spalte 3, Zeile 54; Abbildung 1 *<br>----- | 10-12 | |

Der vorliegende Recherchenbericht wurde für alle Patentansprüche erstellt

| Recherchenort | Abschlußdatum der Recherche | Prüfer |
|---|---|---|
| Den Haag | 19. November 2021 | Reeb, Bertrand |

## ANHANG ZUM EUROPÄISCHEN RECHERCHENBERICHT
## ÜBER DIE EUROPÄISCHE PATENTANMELDUNG NR.

EP 21 18 6368

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

19-11-2021

| Im Recherchenbericht angeführtes Patentdokument | Datum der Veröffentlichung | Mitglied(er) der Patentfamilie | Datum der Veröffentlichung |
|---|---|---|---|
| US 2018024026 A1 | 25-01-2018 | CA 2975685 A1<br>CN 107407613 A<br>EP 3265770 A1<br>JP 2018507415 A<br>JP 2019144275 A<br>KR 20170108086 A<br>SG 11201706318S A<br>US 2018024026 A1<br>WO 2016140736 A1 | 09-09-2016<br>28-11-2017<br>10-01-2018<br>15-03-2018<br>29-08-2019<br>26-09-2017<br>28-09-2017<br>25-01-2018<br>09-09-2016 |
| US 2020225070 A1 | 16-07-2020 | KEINE | |
| US 2016138351 A1 | 19-05-2016 | AU 2015350124 A1<br>BR 112017010359 A2<br>CA 2967813 A1<br>CO 2017005204 A2<br>EA 201791092 A1<br>EP 3221558 A1<br>SG 11201704024S A<br>US 2016138351 A1<br>WO 2016081448 A1 | 01-06-2017<br>03-07-2018<br>26-05-2016<br>31-10-2017<br>30-11-2017<br>27-09-2017<br>29-06-2017<br>19-05-2016<br>26-05-2016 |
| US 2012085434 A1 | 12-04-2012 | KEINE | |
| DE 19615857 C1 | 02-10-1997 | KEINE | |
| US 2009205400 A1 | 20-08-2009 | KEINE | |
| US 5088322 A | 18-02-1992 | KEINE | |
| US 5425815 A | 20-06-1995 | DE 69123153 T2<br>EP 0549674 A1<br>GB 2248188 A<br>JP 3232090 B2<br>JP H06503489 A<br>US 5425815 A<br>WO 9204858 A1 | 03-04-1997<br>07-07-1993<br>01-04-1992<br>26-11-2001<br>21-04-1994<br>20-06-1995<br>02-04-1992 |

EPO FORM P0461

Für nähere Einzelheiten zu diesem Anhang : siehe Amtsblatt des Europäischen Patentamts, Nr.12/82